# EUROPEAN PATENT APPLICATION

(11) **EP 3 029 152 A1**
(43) Date of publication of application: **08.06.2016**
(21) Application number: 14804333.4
(22) Date of filing: 29.05.2014
(51) Int. Cl.: C12Q 1/68, G01N 21/64

(54) **ANGIOTENSIN-CONVERTING ENZYME POLYMORPHISMS FOR PREDICTING OR PROGNOSTICATING THE RESPONSE TO ANTIANGIOGENIC CANCER TREATMENT**

(30) Priority: 29.05.2013 ES 201330789
(71) Applicant: Servicio Andaluz de Salud, 41071 Sevilla (ES); Universidad De Córdoba, 14071 Córdoba (ES)
(72) Inventor: ARANDA AGUILAR, Enrique, E-14004 Córdoba (ES); DE LA HABA RODRÍGUEZ, Juan, E-14004 Córdoba (ES); MARTÍNEZ PEINADO, Antonio, E-14004 Córdoba (ES); RODRÍGUEZ ARIZA, Antonio, E-14004 Córdoba (ES)
(74) Representative: Hoffmann Eitle
(86) International application number: PCT/ES2014/070438
(87) International publication number: WO 2014/191604

(57) **Abstract**

The invention relates to a method and kit for predicting or prognosticating an individual's response to treatment with a thymidine synthesis inhibitor, an alkylation agent and a vascular endothelial growth factor antagonist in a cancer patient, in particular an individual suffering from colon cancer.

SEQUENCE LISTINGS

<110>  Servicio Andaluz de Salud
<110>  Universidad de Córdoba

<120>  Angiotensin-converting enzyme polymorphisms for predicting
 or prognosticating the response to antiangiogenic cancer treatment

<130>  PCT-06858

<160>  8

<170>  PatentIn version 3.5

<210>  1
<211>  50
<212>  DNA
<213>  Homo sapiens

<400>  1
atacagtcac tttttttttt tttttgagac ggagtctcgc tctgtcgccc                50

<210>  2
<211>  100
<212>  DNA
<213>  Homo sapiens

<400>  2
tcccatttct ctagacctgc tgcctataca gtcacttttt tttttttttt gagacggagt      60

ctcgctctgt cgcccataca gtcactttta tgtggtttcg                          100

<210>  3
<211>  1306
<212>  PRT
<213>  Homo sapiens

<400>  3

<210> 4
<211> 4969
<212> DNA
<213> Homo sapiens

<400> 4
agaaggggca gagccgagca ccgcgcaccg cgtcatgggg gccgcctcgg gccgccgggg 60
gccggggctg ctgctgccgc tgccgctgct gttgctgctg ccgccgcagc ccgccctggc 120
gttggacccc gggctgcagc ccggcaactt ttctgctgac gaggccgggg cgcagctctt 180
cgcgcagagc tacaactcca gcgccgaaca ggtgctgttc cagagcgtgg ccgccagctg 240
ggcgcacgac accaacatca ccgcggagaa tgcaaggcgc caggaggaag cagccctgct 300
cagccaggag tttgcggagg cctggggcca gaaggccaag gagctgtatg aaccgatctg 360
gcagaacttc acggacccgc agctgcgcag gatcatcgga gctgtgcgca ccctgggctc 420
tgccaacctg cccctggcta agcggcagca gtacaacgcc ctgctaagca acatgagcag 480
gatctactcc accgccaagg tctgcctccc caacaagact gccacctgct ggtccctgga 540
cccagatctc accaacatcc tggcttcctc gcgaagctac gccatgctcc tgtttgcctg 600
ggagggctgg cacaacgctg cgggcatccc gctgaaaccg ctgtacgagg atttcactgc 660
cctcagcaat gaagcctaca agcaggacgg cttcacagac acgggggcct actggcgctc 720
ctggtacaac tcccccacct tcgaggacga tctggaacac ctctaccaac agctagagcc 780
cctctacctg aacctccatg ccttcgtccg ccgcgcactg catcgccgat acggagacag 840
atacatcaac ctcaggggac ccatccctgc tcatctgctg ggagacatgt gggcccagag 900
ctgggaaaac atctacgaca tggtggtgcc tttcccagac aagcccaacc tcgatgtcac 960
cagtactatg ctgcagcagg gctggaacgc cacgcacatg ttccgggtgg cagaggagtt 1020
cttcacctcc ctggagctct cccccatgcc tcccgagttc tgggaagggt cgatgctgga 1080
gaagccggcc gacgggcggg aagtggtgtg ccacgcctcg gcttgggact tctacaacag 1140
gaaagacttc aggatcaagc agtgcacacg ggtcacgatg gaccagctct ccacagtgca 1200
ccatgagatg ggccatatac agtactacct gcagtacaag gatctgcccg tctccctgcg 1260
tcggggggcc aaccccggct tccatgaggc cattggggac gtgctggcgc tctcggtctc 1320
cactcctgaa catctgcaca aaatcggcct gctggaccgt gtcaccaatg acacggaaag 1380
tgacatcaat tacttgctaa aaatggcact ggaaaaaatt gccttcctgc cctttggcta 1440
cttggtggac cagtggcgct ggggggtctt tagtgggcgt accccccctt cccgctacaa 1500
cttcgactgg tggtatcttc gaaccaagta tcaggggatc tgtcctcctg ttacccgaaa 1560
cgaaacccac tttgatgctg gagctaagtt tcatgttcca aatgtgacac catacatcag 1620
gtactttgtg agttttgtcc tgcagttcca gttccatgaa gccctgtgca aggaggcagg 1680
ctatgagggc ccactgcacc agtgtgacat ctaccggtcc accaaggcag gggccaagct 1740
ccggaaggtg ctgcaggctg gctcctccag gccctggcag gaggtgctga aggacatggt 1800
cggcttagat gccctggatg cccagccgct gctcaagtac ttccagccag tcacccagtg 1860

gctgcaggag cagaaccagc agaacggcga ggtcctgggc tggcccgagt accagtggca 1920
cccgccgttg cctgacaact acccggaggg catagacctg gtgactgatg aggctgaggc 1980
cagcaagttt gtggaggaat atgaccggac atcccaggtg gtgtggaacg agtatgccga 2040
ggccaactgg aactacaaca ccaacatcac cacagagacc agcaagattc tgctgcagaa 2100
gaacatgcaa atagccaacc acaccctgaa gtacggcacc caggccagga agtttgatgt 2160
gaaccagttg cagaacacca ctatcaagcg gatcataaag aaggttcagg acctagaacg 2220
ggcagcactg cctgcccagg agctggagga gtacaacaag atcctgttgg atatggaaac 2280
cacctacagc gtggccactg tgtgccaccc gaatggcagc tgcctgcagc tcgagccaga 2340
tctgacgaat gtgatggcca cgtcccggaa atatgaagac ctgttatggg catgggaggg 2400
ctggcgagac aaggcgggga gagccatcct ccagttttac ccgaaatacg tggaactcat 2460
caaccaggct gcccggctca atggctatgt agatgcaggg gactcgtgga ggtctatgta 2520
cgagacacca tccctggagc aagacctgga gcggctcttc caggagctgc agccactcta 2580
cctcaacctg catgcctacg tgcgccgggc cctgcaccgt cactacgggg cccagcacat 2640
caacctggag gggcccattc ctgctcacct gctggggaac atgtgggcgc agacctggtc 2700
caacatctat gacttggtgg tgcccttccc ttcagccccc tcgatggaca ccacagaggc 2760
tatgctaaag caggctggac cgcccaggag gatgtttaag gaggctgatg atttcttcac 2820
ctccctgggg ctgctgcccg tgcctcctga gttctggaac aagtcgatgc tggagaagcc 2880
aaccgacggg cgggaggtgg tctgccacgc ctcggcctgg gacttctaca acggcaagga 2940
cttccggatc aagcagtgca ccaccgtgaa cttggaggac ctggtggtgg cccaccacga 3000
aatgggccac atccagtatt tcatgcagta caaagactta cctgtggcct tgagggaggg 3060
tgccaacccc ggcttccatg aggccattgg ggacgtgcta gccctctcag tgtctacgcc 3120
caagcacctg cacagtctca acctgctgag cagtgagggt ggcagcgacg agcatgacat 3180
caactttctg atgaagatgg cccttgacaa gatcgccttt atccccttca gctacctcgt 3240
cgatcagtgg cgctggaggg tatttgatgg aagcatcacc aaggagaact ataaccagga 3300
gtggtggagc ctcaggctga agtaccaggg cctctgcccc ccagtgccca ggactcaagg 3360
tgactttgac ccaggggcca agttccacat tccttctagc gtgccttaca tcaggtactt 3420
tgtcagcttc atcatccagt tccagttcca cgaggcactg tgccaggcag ctggccacac 3480
gggccccctg cacaagtgtg acatctacca gtccaaggag gccgggcagc gcctggcgac 3540
cgccatgaag ctgggcttca gtaggccgtg gccggaagcc atgcagctga tcacgggcca 3600
gcccaacatg agcgcctcgg ccatgttgag ctacttcaag ccgctgctgg actggctccg 3660
cacggagaac gagctgcatg gggagaagct gggctggccg cagtacaact ggacgccgaa 3720
ctccgctcgc tcagaagggc ccctcccaga cagcggccgc gtcagcttcc tgggcctgga 3780

cctggatgcg cagcaggccc gcgtgggcca gtggctgctg ctcttcctgg gcatcgccct 3840

gctggtagcc accctgggcc tcagccagcg gctcttcagc atccgccacc gcagcctcca 3900

ccggcactcc cacgggcccc agttcggctc cgaggtggag ctgagacact cctgaggtga 3960

cccggctggg tcggccctgc ccaagggcct cccaccagag actgggatgg gaacactggt 4020

gggcagctga ggacacaccc cacaccccag cccaccctgc tcctcctgcc ctgtccctgt 4080

ccccctcccc tcccagtcct ccagaccacc agccgcccca gccccttctc ccagcacacg 4140

gctgcctgac actgagcccc acctctccaa gtctctctgt gaatacaatt aaaggtcctg 4200

ccctccccat ctgagtctgt gtccctcaca gggaagccag ggacagggac aggctgcttt 4260

cctgcctcct ggcagtcaag tgggtcccgt tactaggttt gttcctccat cctccttcag 4320

gagccgggga ggatccccag agctctgccc cagcacctcc tggcgctggc gcctgtcttc 4380

cctccagccc aggcagcccg ccactgtcct gccaccgcag gcagcccctg tctggcccaa 4440

gcactgaccc acgcggactc tgggaagcag acatcctggg ctgctggcct cacatttcca 4500

ctggcagtgg agcctttccc tgctccacaa atggccaggt ccccccaggg gaaggcttcc 4560

ggctgttatc ggctgcctca gggggcgagt accttggagg gcctgcttca aggagggtgc 4620

cccctggagg gcacacacca gcctagtgct taccttggct cctgcctgta ccagctccat 4680

gactctgctc gggtgaacag ccttggctct cagacagcca ttctaacact gccagtgcag 4740

aggggcctca gacgctggag tgtagcagtg gctgcacctg cacagggatt agctgccagc 4800

agccaccctg ctggcgtccc agcacacacc tcctcactcc ctgcattgga gggagtgtca 4860

ttttaaggga catttttatg acttttatgt gtatgtttat gtagaaattt ggaaaataca 4920

gaaaactgta aagaaaataa aagcccttta tatcaacgtc aagagataa 4969

<210> 5
<211> 732
<212> PRT
<213> Homo sapiens

<400> 5

Met Gly Gln Gly Trp Ala Thr Ala Gly Leu Pro Ser Leu Leu Phe Leu
1               5                   10                  15

Leu Leu Cys Tyr Gly His Pro Leu Leu Val Pro Ser Gln Glu Ala Ser
            20                  25                  30

Gln Gln Val Thr Val Thr His Gly Thr Ser Ser Gln Ala Thr Thr Ser
        35                  40                  45

Ser Gln Thr Thr Thr His Gln Ala Thr Ala His Gln Thr Ser Ala Gln
    50                  55                  60

Ser Pro Asn Leu Val Thr Asp Glu Ala Glu Ala Ser Lys Phe Val Glu
65                  70                  75                  80

Glu Tyr Asp Arg Thr Ser Gln Val Val Trp Asn Glu Tyr Ala Glu Ala
                85                  90                  95

Asn Trp Asn Tyr Asn Thr Asn Ile Thr Thr Glu Thr Ser Lys Ile Leu
            100                 105                 110

Leu Gln Lys Asn Met Gln Ile Ala Asn His Thr Leu Lys Tyr Gly Thr
        115                 120                 125

Gln Ala Arg Lys Phe Asp Val Asn Gln Leu Gln Asn Thr Thr Ile Lys
    130                 135                 140

Arg Ile Ile Lys Lys Val Gln Asp Leu Glu Arg Ala Ala Leu Pro Ala
145                 150                 155                 160

Gln Glu Leu Glu Glu Tyr Asn Lys Ile Leu Leu Asp Met Glu Thr Thr
                165                 170                 175

Tyr Ser Val Ala Thr Val Cys His Pro Asn Gly Ser Cys Leu Gln Leu
            180                 185                 190

Glu Pro Asp Leu Thr Asn Val Met Ala Thr Ser Arg Lys Tyr Glu Asp
        195                 200                 205

Leu Leu Trp Ala Trp Glu Gly Trp Arg Asp Lys Ala Gly Arg Ala Ile
    210                 215                 220

Leu Gln Phe Tyr Pro Lys Tyr Val Glu Leu Ile Asn Gln Ala Ala Arg
225                 230                 235                 240

Leu Asn Gly Tyr Val Asp Ala Gly Asp Ser Trp Arg Ser Met Tyr Glu
                245                 250                 255

Thr Pro Ser Leu Glu Gln Asp Leu Glu Arg Leu Phe Gln Glu Leu Gln
            260                 265                 270

Pro Leu Tyr Leu Asn Leu His Ala Tyr Val Arg Arg Ala Leu His Arg
        275                 280                 285

His Tyr Gly Ala Gln His Ile Asn Leu Glu Gly Pro Ile Pro Ala His
    290                 295                 300

Leu Leu Gly Asn Met Trp Ala Gln Thr Trp Ser Asn Ile Tyr Asp Leu

305                 310                 315                 320

Val Val Pro Phe Pro Ser Ala Pro Ser Met Asp Thr Thr Glu Ala Met
                325                 330                 335

Leu Lys Gln Gly Trp Thr Pro Arg Arg Met Phe Lys Glu Ala Asp Asp
            340                 345                 350

Phe Phe Thr Ser Leu Gly Leu Leu Pro Val Pro Pro Glu Phe Trp Asn
        355                 360                 365

Lys Ser Met Leu Glu Lys Pro Thr Asp Gly Arg Glu Val Val Cys His
    370                 375                 380

Ala Ser Ala Trp Asp Phe Tyr Asn Gly Lys Asp Phe Arg Ile Lys Gln
385                 390                 395                 400

Cys Thr Thr Val Asn Leu Glu Asp Leu Val Val Ala His His Glu Met
                405                 410                 415

Gly His Ile Gln Tyr Phe Met Gln Tyr Lys Asp Leu Pro Val Ala Leu
            420                 425                 430

Arg Glu Gly Ala Asn Pro Gly Phe His Glu Ala Ile Gly Asp Val Leu
        435                 440                 445

Ala Leu Ser Val Ser Thr Pro Lys His Leu His Ser Leu Asn Leu Leu
    450                 455                 460

Ser Ser Glu Gly Gly Ser Asp Glu His Asp Ile Asn Phe Leu Met Lys
465                 470                 475                 480

Met Ala Leu Asp Lys Ile Ala Phe Ile Pro Phe Ser Tyr Leu Val Asp
                485                 490                 495

Gln Trp Arg Trp Arg Val Phe Asp Gly Ser Ile Thr Lys Glu Asn Tyr
            500                 505                 510

Asn Gln Glu Trp Trp Ser Leu Arg Leu Lys Tyr Gln Gly Leu Cys Pro
        515                 520                 525

Pro Val Pro Arg Thr Gln Gly Asp Phe Asp Pro Gly Ala Lys Phe His
    530                 535                 540

Ile Pro Ser Ser Val Pro Tyr Ile Arg Tyr Phe Val Ser Phe Ile Ile
545                 550                 555                 560

Gln Phe Gln Phe His Glu Ala Leu Cys Gln Ala Ala Gly His Thr Gly
                565                 570                 575

Pro Leu His Lys Cys Asp Ile Tyr Gln Ser Lys Glu Ala Gly Gln Arg
            580                 585                 590

Leu Ala Thr Ala Met Lys Leu Gly Phe Ser Arg Pro Trp Pro Glu Ala
        595                 600                 605

Met Gln Leu Ile Thr Gly Gln Pro Asn Met Ser Ala Ser Ala Met Leu
    610                 615                 620

Ser Tyr Phe Lys Pro Leu Leu Asp Trp Leu Arg Thr Glu Asn Glu Leu
625                 630                 635                 640

His Gly Glu Lys Leu Gly Trp Pro Gln Tyr Asn Trp Thr Pro Asn Ser
                645                 650                 655

Ala Arg Ser Glu Gly Pro Leu Pro Asp Ser Gly Arg Val Ser Phe Leu
            660                 665                 670

Gly Leu Asp Leu Asp Ala Gln Gln Ala Arg Val Gly Gln Trp Leu Leu
        675                 680                 685

Leu Phe Leu Gly Ile Ala Leu Leu Val Ala Thr Leu Gly Leu Ser Gln
    690                 695                 700

Arg Leu Phe Ser Ile Arg His Arg Ser Leu His Arg His Ser His Gly
705                 710                 715                 720

Pro Gln Phe Gly Ser Glu Val Glu Leu Arg His Ser
                725                 730

<210>  6
<211>  3264
<212>  DNA
<213>  Homo sapiens

<400>  6
gctcttattg gccaggggac ggtagctgca ggactctgct ctcctgcggc catgggccag      60

ggttgggcta ctgcaggact tcccagcctc ctcttcctgc tgctctgcta cgggcaccct     120

ctgctggtcc ccagccagga ggcatcccaa caggtgacag tcacccatgg gacaagcagc     180

caggcaacaa ccagcagcca gacaaccacc caccaggcga cggcccacca gacatcagcc     240

cagagcccaa acctggtgac tgatgaggct gaggccagca agtttgtgga ggaatatgac     300

cggacatccc aggtggtgtg gaacgagtat gccgaggcca actggaacta caacaccaac     360

atcaccacag agaccagcaa gattctgctg cagaagaaca tgcaaatagc caaccacacc     420

ctgaagtacg gcacccaggc caggaagttt gatgtgaacc agttgcagaa caccactatc 480

aagcggatca taaagaaggt tcaggaccta gaacgggcag cactgcctgc ccaggagctg 540

gaggagtaca acaagatcct gttggatatg gaaaccacct acagcgtggc cactgtgtgc 600

cacccgaatg gcagctgcct gcagctcgag ccagatctga cgaatgtgat ggccacgtcc 660

cggaaatatg aagacctgtt atgggcatgg gagggctggc gagacaaggc ggggagagcc 720

atcctccagt tttacccgaa atacgtggaa ctcatcaacc aggctgcccg gctcaatggc 780

tatgtagatg caggggactc gtggaggtct atgtacgaga caccatccct ggagcaagac 840

ctggagcggc tcttccagga gctgcagcca ctctacctca acctgcatgc ctacgtgcgc 900

cgggccctgc accgtcacta cggggcccag cacatcaacc tggaggggcc cattcctgct 960

cacctgctgg ggaacatgtg ggcgcagacc tggtccaaca tctatgactt ggtggtgccc 1020

ttcccttcag ccccctcgat ggacaccaca gaggctatgc taaagcaggg ctggacgccc 1080

aggaggatgt ttaaggaggc tgatgatttc ttcacctccc tggggctgct gcccgtgcct 1140

cctgagttct ggaacaagtc gatgctggag aagccaaccg acgggcggga ggtggtctgc 1200

cacgcctcgg cctgggactt ctacaacggc aaggacttcc ggatcaagca gtgcaccacc 1260

gtgaacttgg aggacctggt ggtggcccac cacgaaatgg gccacatcca gtatttcatg 1320

cagtacaaag acttacctgt ggccttgagg gagggtgcca accccggctt ccatgaggcc 1380

attggggacg tgctagccct ctcagtgtct acgcccaagc acctgcacag tctcaacctg 1440

ctgagcagtg agggtggcag cgacgagcat gacatcaact ttctgatgaa gatggccctt 1500

gacaagatcg cctttatccc cttcagctac ctcgtcgatc agtggcgctg gagggtattt 1560

gatggaagca tcaccaagga gaactataac caggagtggt ggagcctcag gctgaagtac 1620

cagggcctct gccccccagt gcccaggact caaggtgact ttgacccagg ggccaagttc 1680

cacattcctt ctagcgtgcc ttacatcagg tactttgtca gcttcatcat ccagttccag 1740

ttccacgagg cactgtgcca ggcagctggc cacacgggcc ccctgcacaa gtgtgacatc 1800

taccagtcca aggaggccgg gcagcgcctg gcgaccgcca tgaagctggg cttcagtagg 1860

ccgtggccgg aagccatgca gctgatcacg ggccagccca acatgagcgc ctcggccatg 1920

ttgagctact tcaagccgct gctggactgg ctccgcacgg agaacgagct gcatggggag 1980

aagctgggct ggccgcagta caactggacg ccgaactccg ctcgctcaga agggcccctc 2040

ccagacagcg gccgcgtcag cttcctgggc ctggacctgg atgcgcagca ggcccgcgtg 2100

ggccagtggc tgctgctctt cctgggcatc gccctgctgg tagccaccct gggcctcagc 2160

cagcggctct tcagcatccg ccaccgcagc ctccaccggc actcccacgg gccccagttc 2220

ggctccgagg tggagctgag acactcctga ggtgacccgg ctgggtcggc cctgcccaag 2280

ggcctcccac cagagactgg gatgggaaca ctggtgggca gctgaggaca caccccacac 2340

cccagcccac cctgctcctc ctgccctgtc cctgtccccc tcccctccca gtcctccaga 2400

ccaccagccg ccccagcccc ttctcccagc acacggctgc ctgacactga gccccacctc 2460

tccaagtctc tctgtgaata caattaaagg tcctgccctc cccatctgag tctgtgtccc 2520

tcacagggaa gccagggaca gggacaggct gctttcctgc ctcctggcag tcaagtgggt 2580

cccgttacta ggtttgttcc tccatcctcc ttcaggagcc ggggaggatc cccagagctc 2640

tgccccagca cctcctggcg ctggcgcctg tcttccctcc agcccaggca gcccgccact 2700

gtcctgccac cgcaggcagc ccctgtctgg cccaagcact gacccacgcg gactctggga 2760

agcagacatc ctgggctgct ggcctcacat ttccactggc agtggagcct ttccctgctc 2820

cacaaatggc caggtccccc caggggaagg cttccggctg ttatcggctg cctcaggggg 2880

cgagtacctt ggagggcctg cttcaaggag ggtgccccct ggagggcaca caccagccta 2940

gtgcttacct tggctcctgc ctgtaccagc tccatgactc tgctcgggtg aacagccttg 3000

gctctcagac agccattcta acactgccag tgcagagggg cctcagacgc tggagtgtag 3060

cagtggctgc acctgcacag ggattagctg ccagcagcca ccctgctggc gtcccagcac 3120

acacctcctc actccctgca ttggagggag tgtcatttta agggacattt ttatgacttt 3180

tatgtgtatg tttatgtaga aatttggaaa atacagaaaa ctgtaaagaa aataaaagcc 3240

ctttatatca acgtcaagag ataa 3264

<210> 7
<211> 691
<212> PRT
<213> Homo sapiens

<400> 7

Met Gly Gln Gly Trp Ala Thr Ala Gly Leu Pro Ser Leu Leu Phe Leu
1               5                   10                  15

Leu Leu Cys Tyr Gly His Pro Leu Leu Val Pro Ser Gln Glu Ala Ser
            20                  25                  30

Gln Gln Val Thr Val Thr His Gly Thr Ser Ser Gln Ala Thr Thr Ser
        35                  40                  45

Ser Gln Thr Thr Thr His Gln Ala Thr Ala His Gln Thr Ser Ala Gln
    50                  55                  60

Ser Pro Asn Leu Val Thr Asp Glu Ala Glu Ala Ser Lys Phe Val Glu
65                  70                  75                  80

Glu Tyr Asp Arg Thr Ser Gln Val Val Trp Asn Glu Tyr Ala Glu Ala

                85                  90                  95

Asn Trp Asn Tyr Asn Thr Asn Ile Thr Thr Glu Thr Ser Lys Ile Leu
            100                 105                 110

Leu Gln Lys Asn Met Gln Ile Ala Asn His Thr Leu Lys Tyr Gly Thr
        115                 120                 125

Gln Ala Arg Lys Phe Asp Val Asn Gln Leu Gln Asn Thr Thr Ile Lys
    130                 135                 140

Arg Ile Ile Lys Lys Val Gln Asp Leu Glu Arg Ala Ala Leu Pro Ala
145                 150                 155                 160

Gln Glu Leu Glu Glu Tyr Asn Lys Ile Leu Leu Asp Met Glu Thr Thr
                165                 170                 175

Tyr Ser Val Ala Thr Val Cys His Pro Asn Gly Ser Cys Leu Gln Leu
            180                 185                 190

Glu Pro Asp Leu Thr Asn Val Met Ala Thr Ser Arg Lys Tyr Glu Asp
        195                 200                 205

Leu Leu Trp Ala Trp Glu Gly Trp Arg Asp Lys Ala Gly Arg Ala Ile
    210                 215                 220

Leu Gln Phe Tyr Pro Lys Tyr Val Glu Leu Ile Asn Gln Ala Ala Arg
225                 230                 235                 240

Leu Asn Gly Tyr Val Asp Ala Gly Asp Ser Trp Arg Ser Met Tyr Glu
                245                 250                 255

Thr Pro Ser Leu Glu Gln Asp Leu Glu Arg Leu Phe Gln Glu Leu Gln
            260                 265                 270

Pro Leu Tyr Leu Asn Leu His Ala Tyr Val Arg Arg Ala Leu His Arg
        275                 280                 285

His Tyr Gly Ala Gln His Ile Asn Leu Glu Gly Pro Ile Pro Ala His
    290                 295                 300

Leu Leu Gly Asn Met Trp Ala Gln Thr Trp Ser Asn Ile Tyr Asp Leu
305                 310                 315                 320

Val Val Pro Phe Pro Ser Ala Pro Ser Met Asp Thr Thr Glu Ala Met
                325                 330                 335

Leu Lys Gln Gly Trp Thr Pro Arg Arg Met Phe Lys Glu Ala Asp Asp
            340                 345                 350

Phe Phe Thr Ser Leu Gly Leu Leu Pro Val Pro Pro Glu Phe Trp Asn
        355                 360                 365

Lys Ser Met Leu Glu Lys Pro Thr Asp Gly Arg Glu Val Val Cys His
    370                 375                 380

Ala Ser Ala Trp Asp Phe Tyr Asn Gly Lys Asp Phe Arg Ile Lys Gln
385                 390                 395                 400

Cys Thr Thr Val Asn Leu Glu Asp Leu Val Val Ala His His Glu Met
                405                 410                 415

Gly His Ile Gln Tyr Phe Met Gln Tyr Lys Asp Leu Pro Val Ala Leu
            420                 425                 430

Arg Glu Gly Ala Asn Pro Gly Phe His Glu Ala Ile Gly Asp Val Leu
        435                 440                 445

Ala Leu Ser Val Ser Thr Pro Lys His Leu His Ser Leu Asn Leu Leu
    450                 455                 460

Ser Ser Glu Gly Gly Ser Asp Glu His Asp Ile Asn Phe Leu Met Lys
465                 470                 475                 480

Met Ala Leu Asp Lys Ile Ala Phe Ile Pro Phe Ser Tyr Leu Val Asp
                485                 490                 495

Gln Trp Arg Trp Arg Val Phe Asp Gly Ser Ile Thr Lys Glu Asn Tyr
            500                 505                 510

Asn Gln Glu Trp Trp Ser Leu Arg Leu Lys Tyr Gln Gly Leu Cys Pro
        515                 520                 525

Pro Val Pro Arg Thr Gln Gly Asp Phe Asp Pro Gly Ala Lys Phe His
    530                 535                 540

Ile Pro Ser Ser Val Pro Tyr Ile Arg Thr Ala Met Lys Leu Gly Phe
545                 550                 555                 560

Ser Arg Pro Trp Pro Glu Ala Met Gln Leu Ile Thr Gly Gln Pro Asn
                565                 570                 575

Met Ser Ala Ser Ala Met Leu Ser Tyr Phe Lys Pro Leu Leu Asp Trp
            580                 585                 590

Leu Arg Thr Glu Asn Glu Leu His Gly Glu Lys Leu Gly Trp Pro Gln
        595                 600                 605

Tyr Asn Trp Thr Pro Asn Ser Ala Arg Ser Glu Gly Pro Leu Pro Asp
    610                 615                 620

Ser Gly Arg Val Ser Phe Leu Gly Leu Asp Leu Asp Ala Gln Gln Ala
625                 630                 635                 640

Arg Val Gly Gln Trp Leu Leu Leu Phe Leu Gly Ile Ala Leu Leu Val
                645                 650                 655

Ala Thr Leu Gly Leu Ser Gln Arg Leu Phe Ser Ile Arg His Arg Ser
            660                 665                 670

Leu His Arg His Ser His Gly Pro Gln Phe Gly Ser Glu Val Glu Leu
        675                 680                 685

Arg His Ser
    690

<210>  8
<211>  3141
<212>  DNA
<213>  Homo sapiens

<400>  8
gctcttattg gccaggggac ggtagctgca ggactctgct ctcctgcggc catgggccag      60

ggttgggcta ctgcaggact tcccagcctc ctcttcctgc tgctctgcta cgggcaccct     120

ctgctggtcc ccagccagga ggcatcccaa caggtgacag tcacccatgg gacaagcagc     180

caggcaacaa ccagcagcca gacaaccacc caccaggcga cggcccacca gacatcagcc     240

cagagcccaa acctggtgac tgatgaggct gaggccagca agtttgtgga ggaatatgac     300

cggacatccc aggtggtgtg gaacgagtat gccgaggcca actggaacta caacaccaac     360

atcaccacag agaccagcaa gattctgctg cagaagaaca tgcaaatagc caaccacacc     420

ctgaagtacg gcacccaggc caggaagttt gatgtgaacc agttgcagaa caccactatc     480

aagcggatca taaagaaggt tcaggaccta gaacgggcag cactgcctgc ccaggagctg     540

gaggagtaca acaagatcct gttggatatg gaaaccacct acagcgtggc cactgtgtgc     600

cacccgaatg gcagctgcct gcagctcgag ccagatctga cgaatgtgat ggccacgtcc     660

cggaaatatg aagacctgtt atgggcatgg gagggctggc gagacaaggc ggggagagcc     720

atcctccagt tttacccgaa atacgtggaa ctcatcaacc aggctgcccg gctcaatggc     780

tatgtagatg caggggactc gtggaggtct atgtacgaga caccatccct ggagcaagac     840

ctggagcggc tcttccagga gctgcagcca ctctacctca acctgcatgc ctacgtgcgc 900
cgggccctgc accgtcacta cggggcccag cacatcaacc tggaggggcc cattcctgct 960
cacctgctgg ggaacatgtg ggcgcagacc tggtccaaca tctatgactt ggtggtgccc 1020
ttcccttcag ccccctcgat ggacaccaca gaggctatgc taaagcaggg ctggacgccc 1080
aggaggatgt ttaaggaggc tgatgatttc ttcacctccc tggggctgct gcccgtgcct 1140
cctgagttct ggaacaagtc gatgctggag aagccaaccg acgggcggga ggtggtctgc 1200
cacgcctcgg cctgggactt ctacaacggc aaggacttcc ggatcaagca gtgcaccacc 1260
gtgaacttgg aggacctggt ggtggcccac cacgaaatgg gccacatcca gtatttcatg 1320
cagtacaaag acttacctgt ggccttgagg gagggtgcca accccggctt ccatgaggcc 1380
attggggacg tgctagccct ctcagtgtct acgcccaagc acctgcacag tctcaacctg 1440
ctgagcagtg agggtggcag cgacgagcat gacatcaact ttctgatgaa gatggccctt 1500
gacaagatcg cctttatccc cttcagctac ctcgtcgatc agtggcgctg gagggtattt 1560
gatggaagca tcaccaagga gaactataac caggagtggt ggagcctcag gctgaagtac 1620
cagggcctct gccccccagt gcccaggact caaggtgact ttgacccagg ggccaagttc 1680
cacattcctt ctagcgtgcc ttacatcagg accgccatga agctgggctt cagtaggccg 1740
tggccggaag ccatgcagct gatcacgggc cagcccaaca tgagcgcctc ggccatgttg 1800
agctacttca agccgctgct ggactggctc cgcacggaga acgagctgca tggggagaag 1860
ctgggctggc cgcagtacaa ctggacgccg aactccgctc gctcagaagg gcccctccca 1920
gacagcggcc gcgtcagctt cctgggcctg gacctggatg cgcagcaggc ccgcgtgggc 1980
cagtggctgc tgctcttcct gggcatcgcc ctgctggtag ccaccctggg cctcagccag 2040
cggctcttca gcatccgcca ccgcagcctc caccggcact cccacgggcc ccagttcggc 2100
tccgaggtgg agctgagaca ctcctgaggt gacccggctg ggtcggccct gcccaagggc 2160
ctcccaccag agactgggat gggaacactg gtgggcagct gaggacacac cccacacccc 2220
agcccaccct gctcctcctg ccctgtccct gtcccctccc cctcccagtc ctccagacca 2280
ccagccgccc cagccccttc tcccagcaca cggctgcctg acactgagcc ccacctctcc 2340
aagtctctct gtgaatacaa ttaaaggtcc tgccctcccc atctgagtct gtgtccctca 2400
cagggaagcc agggacaggg acaggctgct ttcctgcctc ctggcagtca agtgggtccc 2460
gttactaggt ttgttcctcc atcctccttc aggagccggg gaggatcccc agagctctgc 2520
cccagcacct cctggcgctg gcgcctgtct tccctccagc ccaggcagcc cgccactgtc 2580
ctgccaccgc aggcagcccc tgtctggccc aagcactgac ccacgcggac tctgggaagc 2640
agacatcctg ggctgctggc ctcacatttc cactggcagt ggagcctttc cctgctccac 2700
aaatggccag gtccccccag gggaaggctt ccggctgtta tcggctgcct cagggggcga 2760

gtaccttgga gggcctgctt caaggagggt gccccctgga gggcacacac cagcctagtg 2820

cttaccttgg ctcctgcctg taccagctcc atgactctgc tcgggtgaac agccttggct 2880

ctcagacagc cattctaaca ctgccagtgc agaggggcct cagacgctgg agtgtagcag 2940

tggctgcacc tgcacaggga ttagctgcca gcagccaccc tgctggcgtc ccagcacaca 3000

cctcctcact ccctgcattg gagggagtgt cattttaagg gacattttta tgacttttat 3060

gtgtatgttt atgtagaaat ttggaaaata cagaaaactg taaagaaaat aaaagccctt 3120

tatatcaacg tcaagagata a 3141

## Description

### Field of the Invention

The present invention is in the field of the molecular biology and medicine. Specifically, it relates to a method for obtaining data useful for predicting or prognosticating a response to antiangiogenic treatment in cancer patients, and in particular individuals suffering from colon cancer. The prediction of response is performed by means of *ACE* (angiotensin-converting enzyme) biomarker analysis.

### Background of the Invention

Angiogenesis, or new blood vessel formation from preexisting vessels, is an essential process whereby solid tumors can grow and metastasize. Angiogenesis is mediated by several proangiogenic molecular factors, among which vascular endothelial growth factor (VEGF) stands out. The start of antiangiogenic therapy in cancer began in 2004 with the approval of bevacizumab, a monoclonal antibody targeting VEGF, limiting angiogenesis and tumor growth. The success of this drug in the treatment of several types of solid tumors, including the metastatic colorectal cancer and the non-small cell lung cancer, led in the following years to the approval of sorafenib, sunitinib, and pazopanib, three tyrosine kinase inhibitors (TKIs) with antiangiogenic activity, approved for use in metastatic renal cancer and other highly vascularized solid tumors (Schmidinger et al., 2008. J. Clin. Oncol. 26(32), 5204-5212). Nevertheless, these TKIs inhibit a wide range of kinases in addition to VEGFR, which entails several adverse effects unrelated to efficient blocking of VEGF signaling. As a result, advances have recently been made in the development of second-generation TKIs, which are more selective for VEGFR, such as tivozanib and axitinib, which are currently in different clinical trial phases (Bhargava and Robinson, 2011. Curr. Oncol. Rep. 13(2), 103-11).

In a significant manner, clinical data indicates that antiangiogenic therapies have a greater antitumor effect in patients who develop hypertension during treatment, including progression-free survival (PFS) and overall survival (OS). In a retrospective review study (Wilhelm et al., 2010. J. Clin. Oncol. 28(15), e15047) of 131 metastatic renal carcinoma patients who received antiangiogenic therapy (sunitinib, sorafenib, and bevacizumab), progression of the disease at 12 months occurred in 37% of patients who developed hypertension (63% of all patients) versus 52% in those patients who did not develop hypertension. Accordingly, the mortality rate was 8% in hypertensive patients, compared to 27% in normotensive patients. In a study similar (Scartozzi et al., 2009. Ann. Oncol. 20(2), 227-30) there was a significant correlation between PFS and the development of hypertension in patients of metastatic colorectal cancer treated with bevacizumab. Median PFS was 14.5 months in patients who developed hypertension, versus 3.1 months in those who did not (p=0.04). A retrospective analysis of four clinical trials, in which metastatic renal cancer patients received sunitinib, demonstrated that all the drug efficacy indicators (including PFS) showed a greater benefit in patients who developed hypertension during treatment with this antiangiogenic (Rini et al., 2011. J. Natl. Cancer Inst. 103(9), 763-73).

This data suggests that in the context of the antiangiogenic treatment, there are certain characteristics inherent to the patient's vasculature, which not only help with antitumor treatment efficacy, but are also predisposed to the development of clinically relevant vascular problems. Among these characteristics is a prior endothelial dysfunction or greater dependency on growth factors such as VEGF for maintaining normal vascular homeostasis. In fact, although it plays an important role the tumor angiogenesis, VEGFR signaling also regulates normal vascular permeability and endothelial cell survival. Furthermore, the high incidence of induced hypertension induced by drugs blocking VEGFR signaling suggests that this pathway can play a more important role that what has been recognized up until now in regulating the systemic vascular resistance. Nevertheless, although the mechanisms involved in vascular toxicity exerted by antiangiogenic therapies are not exactly known, the following have been suggested as possible causes: 1) a blockage of the VEGF pathway involves a reduction in the synthesis of endothelial nitric oxide (eNO), which participates in vasodilatation (Hodd et al., 1998. Am. J. Physiol. 274(3), H1054-8), 2) a reduction in the number of small arteries and arterioles due to antiangiogenic action (Noon et al., 1997 J. Clin. Invest. Apr. 15, 99(8), 1873-9) and 3) an increase in rigidity vascular, fundamentally at the large vessel level (Safar ME et al., 2003. Circulation 107(22), 2864-9).

There are two main types of VEGF receptors: VEGFR1 (also known as Flt-1) and VEGFR2 (KDR or Flk-1), although the function of VEGFR1 is not so well known as that of VEGFR2. In vasculature, VEGFR2 regulates cell proliferation through MAPK pathways, migration by means of focal adhesion kinases and cell survival through Akt. In the case of VEGF, it has been described that a given VEGF genotype (VEGF-2578C/A) is associated with a higher probability of therapeutically benefiting from bevacizumab (Schneider et al., 2008. J. Clin. Oncol. 26, 4672-4678; Tan et al., 2009. J. Clin. Oncol. 27, 1342-1343). It has also recently been suggested that polymorphism VEGF-1498 C/T constitutes a possible predictor of the efficacy of adding bevacizumab to first-line treatment with FOLFIRI in metastatic colorectal cancer patients (Loupakis et al., 2011. BMC Cancer 11, 247. doi: 10.1186/1471-2407-11-247). Finally, it is interesting to point out that the polymorphism of a single nucleotide rs9582036-A in VEGFR-1 has proven to be a possible predictor of response to bevacizumab in metastatic pancreatic cancer patients (Lambretchs et al., 2009. Joint ECCO-ESMO Multidisciplinary Congress; 2009 Sep 20-24; Berlin, Germany. Abstract No. 16LBA). Nevertheless, more data confirming the possible role of VEGF and VEGFR-1 polymorphisms as predictors of the response to antiangiogenic treatment in different types of cancer is needed.

VEGFR2 also stimulates endothelial nitric oxide synthase (eNOS) and the subsequent production of nitric oxide (NO). The role of NO in vascular biology is well-known, with a high vasodilating capacity. Therefore, it plays an essential role in regulating blood pressure and therefore in the development of hypertension (Cooke et al., 1997. Annu. Rev. Med. 48(1) 489-509). The NOS3 gene, coding for eNOS, has been widely studied in the search for genetic variants. The only common variant identified in NOS3 entailing amino acid substitution in the mature protein is variant G894T, or Glu298Asp (rs1799983), in which a G>T substitution in exon 7 causes a change from glutamate to aspartate in position 298 of the amino acid sequence of the protein (Casas et al., 2006. Am. J. Epidemiol. 164(10), 921-35). Several SNPs have also been identified in the promoting region of the gene, although none of them coincides with consensus sequences for any known transcription factor of the NOS3 gene. Likewise, variants in the non-coding 3' region of the gene that may affect stability of the resulting mRNA are not known (Casas et al., 2006. Am. J. Epidemiol. 164(10), 921-35). Although it remains to be suitably characterized whether or not the genetic variations of the NOS3 gene have functional consequences, most studies have focused on three supposedly functional variants of this gene: -786T>C (rs2070744), a 27 base pair-repetition in intron 4 (rs61722009) and the change Glu298Asp (rs1799983). Furthermore, a recent meta-analysis indicates the association of these variants in the NOS3 gene with the arterial hypertension (Niu et al., 2011. PLoS One. 6(9), e24266).

On the other hand, it is known that hypertension and the rennin-angiotensin-aldosterone system (RAAS) has an effect on physiological and tumor neoangiogenesis processes (Ager et al., 2008. Carcinogenesis 2008, 29, 1675-1684; Fendrich et al., 2010. Gut, 59 (5), 630-7; Rhodes et al., 2009. Proc. Natl. Acad. Sci. USA 106, 10284-10289). The RAAS is an enzymatic endocrine system that plays a crucial role in the biological regulation of vasoconstriction, sodium retention and release of antidiuretic hormones such as aldosterone. The RAAS comprises a circulating precursor protein, angiotensinogen tetradecapeptide (AGT), synthesized in the liver and kidney which, after actuation of the rennin proteases and the angiotensin-converting enzyme (ACE), it causes the active octapeptide angiotensin II to act on angiotensin receptors 1 and 2 (AGTR1 and AGTR2) located in the membrane of the target cells. It has been described how angiotensin II, by acting in endothelial cells through AGTR1, activates VEGF proliferation and synthesis (Herr et al., 2008. Reproduction 136, 125-130). Furthermore, inhibition of VEGF synthesis through the specific blocking of AGTR1 with losartan has recently been described in breast cancer cells (Napoleone et al., 2011. Thromb Res. Dec 19). It is important to stress that studies conducted by the authors of the present invention have clearly shown that expression of AGTR1 can be a possible predictive factor of the response to neoadjuvant chemotherapy and bevacizumab in breast cancer (Sánchez-Rovira et al., 2010. J. Clin. Oncol. 28, 15 suppl (May 20 Supplement, ASCO Annual Meeting Proceedings), 556).

Prior results indicate in two independent series that those patients with breast cancer who receive antiangiogenic therapy and chemotherapy in contexts of neoadjuvancy and metastatic disease, have higher response rates if their tumors express AGTR1 (Sánchez-Rovira, et al. 2013. Bevacizumab plus preoperative chemotherapy in operable HER2 negative breast cancer: biomarkers and pathologic response. Clin Transl. Oncol.)

A series of polymorphisms related to modifications in RAAS activity and which could therefore modulate activity in hypertension and/or tumor angiogenesis processes either directly or through the effect thereof on the neoplastic cell, have been described. Polymorphism A1166C in the AGTR1 gene is the polymorphism that has been most frequently related to arterial hypertension. Significantly, this polymorphism is associated with processes of breast carcinogenesis (Xi et al., 2011. Breast Cancer Res. Treat. 130(2), 561-8). As regards the AGT gene, polymorphism M235T in exon 2, which is a T>C substitution causing a substitution of a methionine with a threonine in the AGT molecule, entails an increase from 10 to 20% in AGT plasma concentration. In several studies, the TT genotype of this polymorphism has been associated with a higher risk of breast cancer (Xi et al., 2011. Breast Cancer Res. Treat. 130(2), 561-8). Finally, the most common polymorphism in the *ACE* gene consists of the insertion (I) or deletion (D) of a fragment of 287 pb in the intron 16, and is responsible for the variation interindividual in the expression of *ACE* or ACE activity in blood and tissues (Rigat et al., 1990. J.Clin.Invest. 86, 1343-1346).

There are other polymorphisms that increase susceptibility to developing arterial hypertension, as is the case of those polymorphisms related to the G-protein-coupled beta-adrenoceptor system. Adrenergic receptor B1 (ADRB1) is a receptor associated with the G-protein and participates in, among other functions, the release of rennin from renal juxtaglomerular cells, shows polymorphism Gly389Arg, with as functional importance because in a clinical context an Arg389 receptor seems to respond better to beta-blockers (Johnson and Liggett, 2011. Clin. Pharmacol. Ther. 89(3), 366-78). In the case of the ADRB2 receptor, polymorphism Arg16Gly can have an effect on vasodilating responses due to differences in the generation of NO, and in fact it has been proven that the combination thereof with polymorphism Glu298Asp in the NOS3 gene notably increases the risk of developing hypertension (Misono et al., 2009. Hypertens. 27(7), 1377-83). There is also a polymorphism Gln27Glu in the ADRB2 gene which causes an increase in isoprenaline-induced vasodilatation, which suggests a role in the determination of vascular reactivity (Khalaila et al., 2007. Pharmacogenet Genomics. 17(10), 803-11). Finally, several studies have pointed out an association between some polymorphisms of the GNAS1 gene, coding for the G-protein alpha subunit, and the development of hypertension. Specifically, polymorphism T393C in the GNAS1 gene is significantly associated with the development of hypertension and with a poor response to beta-blockers (Yamamoto et al., 2004. Hypertens. Res. 27(12):919-24). Also, a polymorphism of a single nucleotide in the GNB3 gene, coding for G-protein beta3 subunit, (GNB3 825C>T, rs5443) has been associated with arterial hypertension and is related to a variant of this polypeptide which appears by alternative "splincing" (GNB3-S), and which seems to have increases activity (Johnson and Liggett, 2011. Clin. Pharmacol. Ther. 89(3), 366-78).

### Description of the Invention

The authors of the present invention have analyzed the *ACE* gene polymorphism in individuals suffering from colon cancer who have received treatment with chemotherapy (fluoropyrimidines and oxiplatin) along with bevacizumab. They have found differences that allow them to predict and/or prognosticate an individual's response to antiangiogenic treatment in patients with colon cancer. Therefore, the present invention provides a method for obtaining useful data prognosticating an individual's response to antiangiogenic treatment in patients with colon cancer, as well as a kit or device for predicting or prognosticating the response to antiangiogenic treatment.

Therefore, a first aspect of the invention relates to the use of *ACE* gene polymorphism rs1799752 for predicting or prognosticating a human subject's response to treatment with a vascular endothelial growth factor inhibitor (VEGF) in a cancer patient, and in particular an individual suffering from colon cancer. Another aspect of the invention relates to the use of *ACE* gene polymorphism rs1799752 for predicting or prognosticating a human subject's response to treatment with a thymidine synthesis inhibitor, an alkylation agent and a vascular endothelial growth factor inhibitor (VEGF), in particular a subject suffering from cancer. In a preferred embodiment, the cancer is colon cancer. In another preferred embodiment of this aspect of the invention, the thymidine synthesis inhibitor is a pyrimidine analogue. More preferably, the pyrimidine analogue is selected from the list consisting of cytarabine, fluoracil (5-fluoracil or 5-FU), tegafur, carmofur, gemcitabine, capecitabine, azacitidine, decitabine, or any of the corresponding salts, isomers, prodrugs, derivatives or analogues thereof, and the combinations thereof. In another preferred embodiment the alkylation agent is oxaliplatin. In another preferred embodiment, in treatment the growth factor inhibitor is bevacizumab.

In a preferred embodiment of the invention, response to treatment is measured as an overall response rate or rate of progression of the disease.

### Method of the Invention

Another aspect of the invention relates to a method for obtaining useful data, hereinafter first method of the invention, for predicting or prognosticating an individual's response to treatment with a thymidine synthesis inhibitor, an alkylation agent and a vascular endothelial growth factor inhibitor (VEGF), in which the individual suffers from colon cancer, comprising:
a) Obtaining an isolated sample from the individual.
b) Detecting *ACE* gene genetic polymorphism rs1799752 in the isolated biological sample of (a).

In a preferred embodiment of this aspect of the invention, an individual having the *ACE* I/I genotype is classified in the group of individuals presenting a more rapid progression of the disease and a low response rate with respect to treatment.

In a preferred embodiment of this aspect of the invention, an individual having the *ACE* I/D and *ACE* D/D genotype is classified in the group of individuals having a better response rate with respect to treatment and a slower progression of the disease.

In this specification, individual having the *ACE* I/I genotype is understood as being that individual having in polymorphism rs1799752 an insertion (INS) of SEQ ID NO: 1, in homozygosis.

In this specification, individual having the *ACE* I/D genotype is understood as being that individual having in polymorphism rs1799752 in heterozygosis, the loss (deletion -DEL-) of the insertion in one of the alleles. In this specification, individual having the *ACE* D/D genotype is understood as being that individual that does not have the insertion in any of the alleles.

In another preferred embodiment of this aspect of the invention, the thymidine synthesis inhibitor is a fluoropyrimidine. In another preferred embodiment, the alkylation agent is oxaliplatin. In another preferred embodiment, in treatment the growth factor inhibitor is bevacizumab. In another preferred embodiment, the cancer is colon cancer.

Preferably the method of the invention comprises determining the *ACE* gene polymorphism; even more preferably, the *ACE* gene polymorphism is rs1799752.

The nucleotide sequence of the polymorphism is:
rs 1799752
   ATACAGTCACTTTTTTTTTTTTTTTGAGACGGAGTCTCGCTCTGTCGCCC (SEQ ID NO: 1)
ACE insertion sequence, with adjacent regions (SEQ ID NO: 2)

TCCCATTTCTCTAGACCTGCTGCCT[-/ATACAGTCACTTTTTTTTTTTTT
TTGAGACGGAGTCTCGCTCTGTCGCCC]ATACAGTCACTTTTATGTGGTTTCG

Step (b) of the method described above can be completely or partially automated, for example, by means of robotic equipment for detecting polymorphisms.

An "isolated biological sample" includes, without being limited to, cells, tissues and/or biological fluids from an organism obtained by means of any method known by a person skilled in the art. Preferably, the isolated biological sample from an individual of step (a) is a blood sample. As it is used in the description, the term "individual" refers to animals, preferably mammals, and more preferably humans.

The term "individual" does not seek to be limiting in any aspect, and said individual can be of any age, sex and physical condition.

*ACE* gene polymorphism rs1799752 can be analyzed by any means known in the state of the art. The authors of the present invention have proven that the analysis of this polymorphism allows predicting or prognosticating an individual's response to antiangiogenic treatment in patients suffering from colon cancer. There are various SNP genotyping methods, any of such methods being applicable in the present invention. For example, without limitation, it can be detected using restriction enzymes or by simple PCR sequencing, ASO and DNA microarray hybridization probes or spheres. For example, SNP amplification methods include PCR, the Invader method, rolling circle DNA amplification, etc. As allelic discrimination methods it is possible to use, without limitation, allele-specific hybridization, ligation of allele-specific oligonucleotides, minisequencing or extension probes (SBE, Single Base Extension), invasive allele-specific cleavage (Invader), etc. According to the detection format, it is possible to mention, also without limitation, the use of Taqman probes, molecular beacons, scorpion, etc. Regarding reactions on a solid support, there are, for example, DNA microarrays and biochips, or other methods using particles, such as Luminex 100, BeadArray, etc. There are also mass detection methods, such as pyrosequencing, miniature electrophoresis, d-PCR or ion torrent sequencing. Preferably, *ACE* gene polymorphism rs1799752 is analyzed by SimpleProbe.

Characteristics of the ACE enzyme are described below:
ACE (also known as peptidyl dipeptidase A, DCP, ICH, ACE1, DCP1, CD143 or MVCD3) is a metallopeptidase-type enzyme that converts angiotensin I into the active angiotensin II peptide and inactivates bradykinin. The complete amino acid sequence of the enzyme has 1308 residues, and when the gene contains the polymorphism there is a loss due to deletion (ACE D/D) or an insertion (ACE I/I). From the functional viewpoint, this modification determines for the case of the ACE D/D genotype an increase in ACE levels and an increase in angiotensin II levels; this is correlated with a higher probability of developing AHT (arterial hypertension).

In the context of the present invention, *ACE* is also defined by a sequence of nucleotides or polynucleotides, which constitutes the protein coding sequence included in SEQ ID NO: 3, SEQ ID NO: 5, SEQ ID NO: 7 and/or included in the UniProtKB database with number P12821 (UniProtKB), and it would comprise various variants from:
a) nucleic acid molecules coding for a polypeptide comprising the amino acid sequence of SEQ ID NO: 3, SEQ ID NO: 5: SEQ ID NO: 7 and/or included in the UniProtKB database with number P12821 (UniProtKB),
b) nucleic acid molecules the complementary chain of which hybridizes with the polynucleotide sequence of a),
c) nucleic acid molecules the sequence of which differs from a) and/or b) due to genetic code degeneration,
d) nucleic acid molecules coding for a polypeptide comprising the amino acid sequence with an identity of at least 80%, 90%, 95%, 98% or 99% with SEQ ID NO: 3, SEQ ID NO: 5, SEQ ID NO: 7 and/or included in the UniProtKB database with number P12821 (UniProtKB), and in which the polypeptide coded by said nucleic acids possesses the activity and the characteristics structural of the protein *ACE.* Among said nucleic acid molecules, the nucleic acid molecule can be the one included in sequence SEQ ID NO: 4, SEQ ID NO: 8 and SEQ ID NO: 8.

In another preferred embodiment, the determination of the polymorphism is performed by means of a PCR method. For example, but without limitation, can be used the RFLP (Restriction Fragment Site Polymorphism), AFLP (Amplified Fragment Length Polymorphism), and RAPD (Random Amplified Polymorphic DNA) techniques, methods using probes, or by direct sequencing.

In another preferred embodiment, the thymidine synthesis inhibitor is a pyrimidine analogue. In another preferred embodiment of this aspect of the invention, the analogues of pyrimidine are selected from the list consisting of cytarabine, fluoracil (5-fluoracil or 5-FU), tegafur, carmofur, gemcitabine, capecitabine, azacitidine, decitabine, or any of the corresponding salts, isomers, prodrugs, derivatives or analogues thereof, and the combinations thereof.

In another preferred embodiment, the alkylation agent is oxaliplatin.

In another preferred embodiment, the vascular endothelial growth factor antagonist is selected from sunitinib, sorafenib, bevacizumab, ranibizumab and pegaptanib, or any of the combinations thereof.

### Kit or Device of the Invention

Another aspect of the present invention relates to a kit or device, hereinafter kit of the invention, comprising the tools needed for detecting the *ACE* gene genotype for predicting or prognosticating an individual's response to treatment with a thymidine synthesis inhibitor, a growth factor inhibitor and an alkylation agent, in particular in an individual suffering from cancer. In a preferred embodiment, the cancer is colon cancer. In another preferred embodiment of this aspect of the invention, the thymidine synthesis inhibitor is a pyrimidine analogue. More preferably, the pyrimidine analogue is selected from the list consisting of cytarabine, fluoracil (5-fluoracil or 5-FU), tegafur, carmofur, gemcitabine, capecitabine, azacitidine, decitabine, or any of the corresponding salts, isomers, prodrugs, derivatives or analogues thereof, and the combinations thereof. In another preferred embodiment the alkylation agent is oxaliplatin. In another preferred embodiment, in treatment the growth factor inhibitor is bevacizumab.

The kit can furthermore include, without any type of limitation, buffers, protein extraction solutions, agents for preventing contamination, protein degradation inhibitors, etc.

On the other hand, the kit can include all the carriers and containers necessary for the start up and optimization thereof. Preferably, the kit further comprises instructions for carrying out the methods of the invention.

It is also possible for the oligonucleotide/oligonucleotides or probes to be immobilized in stains on a surface (preferably solid surface). In one of its embodiments, the kit comprises a microarray, or microarray of the invention. An RNA microarray is an array on a solid substrate (usually a glass slide or a thin-film silicon cell) evaluating large amounts of different RNAs that are detectable by means of specific probes immobilized on stains on a solid substrate. Each stain contains a specific nucleic acid sequence, which normally corresponds to a specific DNA sequence. Although the number of stains is not limited in any way, there is a preferred embodiment in which the microarray is customized for the methods of the invention.

In a preferred embodiment of the invention, the kit or device comprises the tools needed for detecting *ACE* gene polymorphism rs1799752.

Another aspect of the invention relates to the use of the kit or device for predicting or prognosticating an individual's response to treatment with a thymidine synthesis inhibitor, an alkylation agent and a growth factor inhibitor vascular endothelium (VEGF) in a cancer patient. In a preferred embodiment, the cancer is colon cancer. In another preferred embodiment of this aspect of the invention, the thymidine synthesis inhibitor is a pyrimidine analogue. More preferably, the pyrimidine analogue is selected from the list consisting of cytarabine, fluoracil (5-fluoracil or 5-FU), tegafur, carmofur, gemcitabine, capecitabine, azacitidine, decitabine, or any of the corresponding salts, isomers, prodrugs, derivatives or analogues thereof, and the combinations thereof. In another preferred embodiment, the alkylation agent is oxaliplatin. In another preferred embodiment, in treatment the growth factor inhibitor is bevacizumab.

Another aspect of the invention relates to a computer-readable storage medium comprising program instructions capable of making a computer carry out the steps of any of the methods of the invention.

Another aspect of the invention relates to a transmissible signal comprising program instructions capable of making a computer carry out the steps of any of the methods of the invention.

The terms "polynucleotide" and "nucleic acid" are used interchangeably herein, in reference to a polymeric forms of nucleotides of any length, both ribonucleotides (RNA) and deoxyribonucleotides (DNA). The terms "amino acid sequence", "peptide", "oligopeptide", "polypeptide" and "protein" are interchangeably used herein, and they refer to a polymeric form of amino acids of any length, which can be coding or non-coding, chemically or biochemically modified amino acids.

Throughout the description and claims the word "comprises" and its variants do not seek to exclude other technical features, additives, components or steps. For the person skilled in the art, other objects, advantages and features of the invention.

### Description of the Drawings

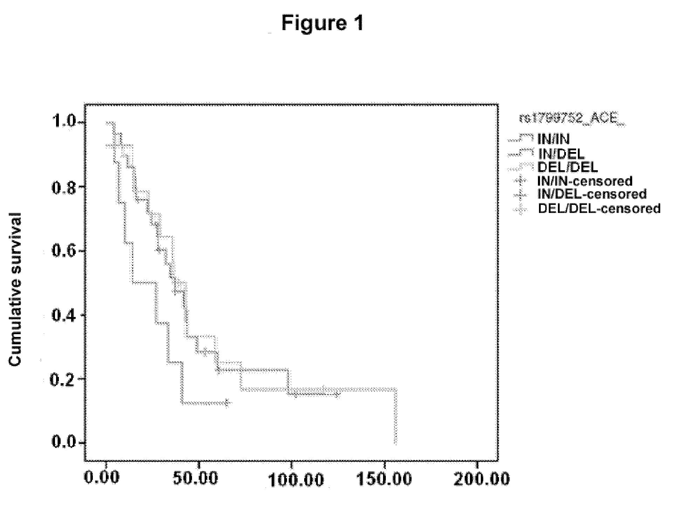

Figure 1 shows the association between the analyzed ACE gene genotypes and survival.

### EXAMPLES OF THE INVENTION

The following example is provided by way of illustration and is not intended to be limiting of the present invention.

### Materials and Methods

The samples (total blood on EDTA + serum) are sent to the molecular genetics section and labeled in an unmistakable manner as participants in the experiment. Serum aliquots are then stored and total blood aliquots separated for DNA extraction.

DNA extraction is carried out on these latter aliquots by means of a robotic method based on paramagnetic particles (MagNAPure LC, from Roche Diagnostics) with MagnaPure LC DNA isolation kit I. Based on 200-400 µl of blood, this method assures obtaining high-quality DNA, free of impurities that may degrade it, in an amount sufficient for subsequent processes (100 µl) and with concentrations (between 10 and 50 ng/µl) that are more than sufficient for that purpose. At least 2 aliquots are always extracted.

The decision was made to perform detection of the different polymorphisms by means of using sensitive and specific probes called SimpleProbe, by means of analysis of the melt curves, in a real-time thermocycler called LigthCycler 480, from Roche Diagnostics, based on 96-well plates. Due to operativity issues, the decision was made to immobilize all the oligonucleotides on the mentioned plates in a format corresponding to 12 SNP x 8 patients (synthesized and immobilized by TIB-MoIBiol).

By using a master mixture which includes the polymerase, buffer, dNTPs, and which keeps the magnesium separate, to activate the reaction only when desired (LightCycler 480 DNA master Hybprobe), the different DNAs were amplified in a final volume of 20 µl from about 50 ng and with a final concentration of 3 mM Mg++. The thermocycling protocol was as follows:

**Table 1. Parameters used in the protocol of thermocycling.**

| Parameter | Denaturation | Thermocycling | | | Melting | | | Cooling |
|---|---|---|---|---|---|---|---|---|
| Type of analysis | None | Quantification | | | Melt curves | | | None |
| Cycles | 1 | 45 | | | 1 | | | 1 |
| Temp. in °C | 95 | 95 | 60 | 72 | 95 | 40 | 75 | 40 |
| Time | 10min | 10s | 10s | 15s | 30s | 2min | 0 | 30s |
| Ramp (°C/s) | 4.4 | 4.4 | 2.2 | 4.4 | 4.4 | 1.5 | NA | 1.5 |
| Acquisition | None | None | Simple | None | None | None | Continuous | None |

The different SNPs analyzed varied from the beginning to the end (www.ncbi.nlm.nih.gov/projects/SNP/)

**Table 2. Listing of initially analyzed SNPs.**

| Initial design | |
|---|---|
| Name | SNPs |
| VEGF -2578 C/A | rs699947 |
| VEGF -1498 C/T | rs833061 |
| VEGFR-1, intron 29 | rs9582036 |
| ACE INS/DEL intron 16 | rs1799752 |
| AGTR1 1166 A/C | rs5186 |
| AGT M235T | rs699 |
| ADRB1 Gly389Arg | rs1801253 |
| ADRB2 Gln27Glu | rs1042714 |
| ADRB2 Gly16Arg | rs1042713 |
| ADRB3 Trp64Arg | rs4994 |
| GNAS 393 T>C | rs62205366 |
| GNB3 825 C>T | rs5443 |

**Table 3. Listing of SNPs that were finally analyzed.**

| Initial design | |
|---|---|
| Name | SNPs |
| VEGF -2578 C/A | rs699947 |
| VEGF -1498 C/T | rs833061 |
| VEGFR-1, intron 29 | rs9582036 |
| ACE INS/DEL intron 16 | rs1799752 |
| AGTR1 1166 A/C | rs5186 |
| AGT M235T | rs699 |
| ADRB1 Gly389Arg | rs1801253 |
| ADRB2 Gln27Glu | rs1042714 |
| ADRB2 Gly16Arg | rs1042713 |
| GNB3 825 C>T | rs5443 |
| NOS3 -786T>C | rs2070744 |
| NOS3 Glu298Asp | rs 1799983 |

SNP rs4994 (in ADRB3) and rs62205366 (in GNAS) were removed from the initial design, the former due to the scarce information that it provided (perhaps due to how rare the least frequent allele is, in the order of 0.09), and the latter because the technical solution chosen does not produce clearly differentiable genotypes in this polymorphism.

Two very interesting polymorphisms in NOS are also included in the final design given their possible effect on vascular dynamics and their possible interactions with those of VEGF.

### Patients/Selection Criteria

Patients that complied with the following criteria were selected:
- Over 18 years of age,
- Signed informed consent
- Histological diagnosis of breast carcinoma (BC) or colorectal carcinoma (CRC).
- Advanced stage, presence of metastatic disease confirmed by an imaging methodology.
- Having received or receiving treatment with antiVEGF monoclonal therapy (bevacizumab) at the dose recommended in the summary of product characteristics
- Availability for follow-up and for obtaining clinical data and biological samples (blood and paraffin-coated tumor material)

Up until the date of the present report, 102 patients comply with these selection criteria, out of which patients 64 suffer from CRC and 38 suffer from BC.

As activity variables to be correlated with each of the polymorphisms:

### 1.- Maximum Response obtained with treatment

Rescist 1.1 Criteria (Response Evaluation Criteria in Solid Tumors), are internationally accepted criteria used to verify if a therapy works and to define if the patient with cancer is suitably responding to treatment.

### 2- Time of Treatment Duration.

Time in weeks going from the start date to the end date of treatment with bevacizumab.

### RESULTS

### SNP ACE INS/DEL Intron 16

**Table 4. Maximum response obtained with treatment depending on SNP ACE INS/DEL Intron 16**

| SNPs | Pro | SD | PR | CR | NA | Chi-square/p |
|---|---|---|---|---|---|---|
| Total n:102 | | | | | | |
| ACE | | | | | | |
| INS/DEL intron 16 | 4(40.0%) | 1(10.0%) | 4(40.0%) | 1(10.0%) | 0(0%) | |
| IN/IN | 5(11.1%) | 20(44.4%) | 13(28.9%) | 6(13.3%) | 1(2.2%) | |
| IN/DEL DEL/DEL | 3(12.5%) | 8(33.3%) | 10(41.7%) | 2(8.3%) | 1(4.2%) | 9.159/ 0.329 |
| Colon n:49 | | | | | | |
| ACE | | | | | | |
| INS/DEL intron 16 | 3(42.9%) | 0(0.0%) | 3(42.9%) | 1(14.3%) | 0(0%) | |
| IN/IN | 2(6.9%) | 13(44.8%) | 9(31.0%) | 4(13.8%) | 1(3.4%) | |
| IN/DEL | 0(0%) | 4(30.8%) | 8(61.5%) | 0(0%) | 1(7.7%) | 16.887/ 0.031 |
| DEL/DEL | | | | | | |
| Mama n:30 | | | | | | |
| ACE | | | | | | |
| INS/DEL intron 16 | 1(33.3%) | 1(33.3%) | 1(33.3%) | 0(0%) | 0(0%) | |
| IN/IN | 3(18.8%) | 7(43.8%) | 4(25.0%) | 2(12.5%) | 1(3.4%) | |
| IN/DEL | 3(27.3%) | 4(33.3%) | 2(18.2%) | 2(18.2%) | 1(7.7%) | 1.347/ 0.969 |
| DEL/DEL | | | | | | |

| | | | | | | |
|---|---|---|---|---|---|---|
| Pro: Progression, SD: Stable Disease, PR: Partial Response, CR: Complete Response, NA: Response Not Assessable | | | | | | |

**Table 5. Time of treatment duration depending on SNP ACE INS/DEL Intron 16.**

| SNPs | Median (sem) | CI95% | Chi-Square/p |
|---|---|---|---|
| Total n:102 | | | |
| ACE | | | |
| INS/DEL intron 16 | 14.00 | 0-36 | |
| IN/IN | 34.71 | 22-47 | |
| IN/DEL | 42.57 | 31-53 | 2.858/0.240 |
| DEL/DEL | | | |
| Colon | | | |
| ACE | | | |
| INS/DEL intron 16 | 14 | 0-37 | |
| IN/IN | 37 | 23-50 | |
| IN/DEL | 36 | 24-47 | |
| DEL/DEL | | | |
| Mama | | | |
| ACE | | | |
| INS/DEL intron 16 | 12.2 | 0-69 | |
| IN/IN | 32.1 | 34-59 | |
| IN/DEL | 46.8 | 14-69 | 3.396/0.18 |
| DEL/DEL | | | |

### Results related to polymorphism rs1799752

For the studied series, the median time to progression is 37 weeks (CI95: 28.7-45.23) and the overall response rate obtained is 73.4% (CR: 9.4%, PR: 35.9%, SD: 28.1 %).

With respect to the polymorphism under study, i.e., *ACE* I/D, has been analyzed for now in 49 patients, 7 of which are I/I, 29 I/D and 13 D/D. In relation to the overall response rate found depending on genotype, the following can be seen: ACE I/I: 57.2%, ACE I/D: 93.1% and ACE D/D: 100%), X²: 16.887, p: 0.031. As regards time to progression, this was less for the In/In genotype. (ACE I/I: 14 weeks, ACE I/D: 37 weeks and ACE D/D: 36 weeks) p: n.s.

## Claims

1. Use of *ACE* gene genetic polymorphism rs1799752, for predicting or prognosticating an individual's response to treatment with a thymidine synthesis inhibitor, an alkylation agent and a vascular endothelial growth factor antagonist in a cancer patient.

2. Use of the *ACE* gene genetic polymorphism according to the preceding claim, where the response to treatment is measured as overall response rate or rate of progression of the disease.

3. Use of the *ACE* gene genetic polymorphism according to either of claims 1-2, where the cancer is colon cancer.

4. A method for obtaining data useful for predicting or prognosticating an individual's response to treatment with a thymidine synthesis inhibitor, an alkylation agent and a vascular endothelial growth factor inhibitor, wherein the individual suffers from cancer, comprising:
a) obtaining an isolated biological sample from an individual,
b) detecting *ACE* gene genetic polymorphism rs1799752 in the isolated biological sample of (a).

5. The method according to the preceding claim, which further comprises:
c) classifying an individual having genotype ACE I/I in the group of individuals presenting a more rapid progression of the disease and a low response rate with respect to treatment.

6. The method according to either of claims 4-5, which further comprises:
d) classifying an individual having the ACE I/D and ACE D/D genotype in the group of individuals having a better response rate with respect to treatment and a slower progression of the disease.

7. The method according to any of claims 4-6, where the individual suffers from colon cancer.

8. The method according to the claims 4-7, where the determination of the polymorphism is performed by means of a PCR method.

9. The method according to any of claims 4-8, where the thymidine synthesis inhibitor is a pyrimidine analogue.

10. The method according to the preceding claim, where the pyrimidine analogue is selected from the list consisting of cytarabine, fluoracil (5-fluoracil or 5-FU), tegafur, carmofur, gemcitabine, capecitabine, azacitidine, decitabine, or any of the corresponding salts, isomers, prodrugs, derivatives or analogues thereof, and the combinations thereof.

11. The method according to any of claims 4-10, where the alkylation agent is oxaliplatin.

12. The method according to any of claims 4-11, where the vascular endothelial growth factor antagonist is selected from sunitinib, sorafenib, bevacizumab, ranibizumab and pegaptanib, or any of the combinations thereof.

13. A kit or device for predicting or prognosticating an individual's response to treatment with a thymidine synthesis inhibitor, an alkylation agent and a vascular endothelial growth factor antagonist, wherein the individual suffers from cancer, comprising the tools needed for detecting *ACE* gene polymorphisms.

14. The kit or device according to the preceding claim, where the *ACE* gene polymorphism is rs1799752.

15. The kit or device according to either of claims 13-14, where the individual suffers from colon cancer.

16. Use of a kit or device according to any of claims 13-15 for predicting or prognosticating an individual's response to treatment with a thymidine synthesis inhibitor, an alkylation agent and a vascular endothelial growth factor antagonist in a cancer patient.
